(19) Europäisches Patentamt
European Patent Office
Office européen des brevets

(11) **EP 1 675 530 B1**

(12) **EUROPÄISCHE PATENTSCHRIFT**

(45) Veröffentlichungstag und Bekanntmachung des Hinweises auf die Patenterteilung:
**31.03.2010 Patentblatt 2010/13**

(21) Anmeldenummer: **04790014.7**

(22) Anmeldetag: **19.10.2004**

(51) Int Cl.:
*A61F 2/16* (2006.01)      *G02C 7/14* (2006.01)

(86) Internationale Anmeldenummer:
**PCT/DE2004/002328**

(87) Internationale Veröffentlichungsnummer:
**WO 2005/039451 (06.05.2005 Gazette 2005/18)**

(54) **INTRAOKULARE LINSENEINRICHTUNG ZUR VERBESSERUNG DES SEHVERMÖGENS BEI NETZHAUTERKRANKUNGEN**

INTRAOCULAR LENS DEVICE FOR IMPROVING VISION IN CASE OF RETINOPATHY

ENSEMBLE LENTILLE INTRAOCULAIRE POUR AMELIORER LA VISION EN CAS DE RETINOPATHIE

(84) Benannte Vertragsstaaten:
**AT BE BG CH CY CZ DE DK EE ES FI FR GB GR HU IE IT LI LU MC NL PL PT RO SE SI SK TR**

(30) Priorität: **20.10.2003 DE 10349254**

(43) Veröffentlichungstag der Anmeldung:
**05.07.2006 Patentblatt 2006/27**

(73) Patentinhaber: **Philipps-Universität Marburg**
**35032 Marburg (DE)**

(72) Erfinder:
• **SEL, Saadettin**
**06120 Halle (Saale) (DE)**
• **HEBER, Jörg**
**London N12 8TW (GB)**

(74) Vertreter: **Buchhold, Jürgen et al**
**Patentanwälte Olbricht & Buchhold**
**Am Weinberg 15**
**35096 Weimar/Lahn (DE)**

(56) Entgegenhaltungen:
EP-A- 1 475 055        WO-A-98/05272
WO-A-2005/011531       US-A- 4 581 031
US-A- 4 828 558        US-A- 5 089 023
US-A- 5 203 788        US-A- 5 489 302
US-A- 5 712 721

**Beschreibung**

**[Stand der Technik]**

Die Erfindung betrifft eine intraokulare Linseneinrichtung, die zur Verbesserung des Sehvermögens bei Netzhaut- und insbesondere Makulaerkrankungen dient.

Häufige Ursachen für Sehbeeinträchtigungen beim Menschen sind krankhafte Veränderungen des Augenhintergrundes (Netzhaut, Aderhaut und Lederhaut). Diese Veränderungen können zur Herabsetzung der Netzhautfunktion führen. Die Aufgabe der Netzhaut ist die neurosensorische Bearbeitung und Weiterleitung des einfallenden Lichtreizes. An den pathologisch veränderten Netzhautarealen ist das Sehen beeinträchtigt bis unmöglich, so dass es an diesen Stellen zu Gesichtsfeldausfällen kommen kann.

Das normale Auge fokussiert mit Hilfe der brechenden Medien (Hornhaut und Linse) parallel einfallende Lichtstrahlen in die Makula. Die Makula ist ein Netzhautareal am hinteren Pol des Auges mit der höchsten optischen Auflösung. Bei krankhafter Veränderung der Makula kann es zu einer erheblichen Herabsetzung des Sehvermögens kommen. Die betroffenen Personen können keine üblichen Tätigkeiten wie Lesen oder Fahren ausführen, da die Lichtstrahlen durch die natürliche biologische Linse auf die krankhaft veränderte Makula projiziert werden. Dadurch ist es für die Patienten unmöglich ein fixiertes Objekt zu erkennen. In den meisten Krankheitsfällen ist nicht die gesamte Netzhaut bzw. Makula beschädigt, sondern weist noch gesunde Bereiche auf. Deshalb könnte das Sehen durch Ablenkung der Lichtstrahlen auf diese gesunden Netzhautstellen mit Hilfe einer intraokularen Linseneinrichtung erheblich verbessert werden.

Es sind bisher mehrere intraokulare Linseneinrichtungen bekannt, die zur Verbesserung des Sehvermögens bei Makulaerkrankungen vorgesehen sind.

Die US-amerikanische Patentschrift US-A-4581031 beschreibt eine intraokulare Linseneinrichtung zur Verbesserung der Sicht bei Patienten mit Verlust des zentralen Gesichtsfeldes.

Dort handelt es sich um eine Linseneinrichtung zum Verschieben von Netzhautbildern mittels eines prismatischen Keils zu einem funktionierenden Teil der Netzhaut. In der Patentschrift EP 0897293 B1 wurde diese Erfindung verbessert, indem ein Paar von intraokulären Linsen verwendet wird, welche das Sehen von Zielen in verschiedenen Entfernungen ermöglichen.

Zur Verschiebung des Brennpunktes auf der Netzhaut wurde ebenfalls ein prismatischer Keil verwendet, der aus einem Material mit einem hohen Brechungsindex im Bereich von 1,5-1,6 besteht.

Die Offenlegungsschrift DE 19751503 A1 zeigt eine prismatische intraokulare Linse, die zum Ersatz der menschlichen Linse im Auge mit integriertem konzentrischen Prisma zur Verbesserung des Sehvermögens nach Kataraktoperationen bei Patienten mit Erkrankungen der zentralen Netzhautgebiete (z.B. der Makula) eingesetzt werden kann. Da die Vorrichtungen ins Auge eingeführt werden, muss gewährleistet werden, dass sie nicht verrutschen. Dies wird nach dem Stand der Technik mittels Halterungsteilen (Haptiken) erreicht. Man kann aber auch andere Fixierungsvorrichtungen verwenden, die das Verrutschen verhindern.

Die vorgenannten Vorrichtungen aus dem Stand der Technik weisen folgende Nachteile auf:

1. Die Vorrichtungen aus US-A-4581031, DE 19751503 A1 und EP 0897293 B1 müssen voluminös sowie starr und damit schwer ausgelegt sein, um eine optische Korrektur-Wirkung in Form einer Verschiebung des Brennpunktes zu bewirken. Zudem müssen für eine ausreichende Verschiebung des Brennpunktes Materiallien mit relativ hohen Brechungsindizes verwendet werden (nach dem Stand der Technik etwa 1,5 bis 1,6).

2. Das Sehvermögen wird durch die Defokussierung (DE 19751503 A1) nur zu einem sehr geringen Teil wiederhergestellt. Die kreisförmige Brennzone führt lediglich zu einer Aufhellung des Gesichtsfeldes.

Damit sind diese Vorrichtungen nur bei einer kleinen Anzahl von Patienten anwendbar und führen bei diesen Patienten zu nur mäßiger optischer Verbesserung bei sehr schlechtem Tragekomfort.

Als nächstliegender Stand der Technik wird die US 5,089,023 A angesehen. Diese offenbart eine Linseneinrichtung zur Implantation ins Auge. Diese Linseneinrichtung umfasst ein konvexes Linsenelement, welches Segmente mit keilförmigen Ausnehmungen aufweist.

Von Nachteil hierbei ist, dass eine solche Vorrichtung relativ voluminös und entsprechend schwer ist.

**[Aufgabe der Erfindung]**

Aufgabe der Erfindung ist es daher, eine Linseneinrichtung zu schaffen, die eine kleinere Gesamtgröße aufweist und das Sehvermögen des erkrankten Menschen höchstmöglich verbessert, wozu die Linseneinrichtung möglichst weit an das Krankheitsbild anpassbar sein sollte.

Durch die gewünschte Größenreduktion können operationsbedingte Komplikationen bedeutend verringert und die Genesungszeit der Patienten verkürzt werden, da der Chirurg einen kürzeren Schnitt zum Einführen der Linse in das Auge benötigt. Insbesondere durch die Faltbarkeit der intraokulären Optik kann die Schnittlänge nochmals verringert werden.

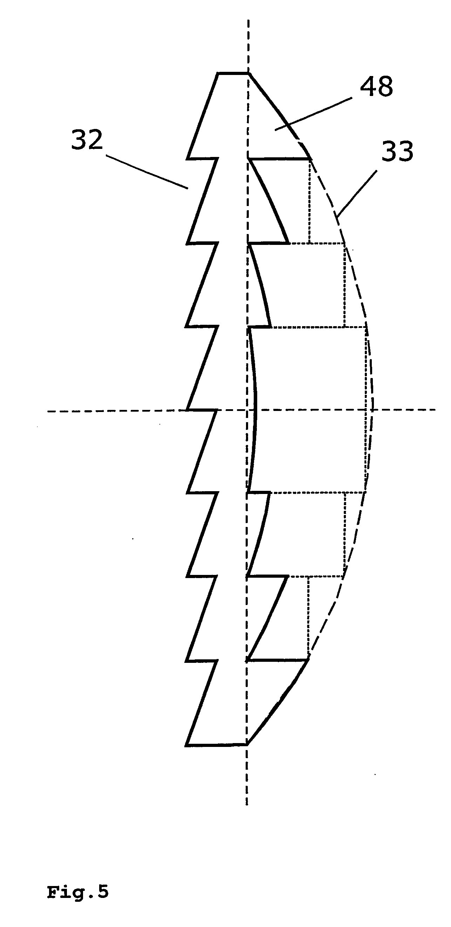

Die gestellte Aufgabe wird erfindungsgemäß durch eine Linseneinrichtung nach Anspruch 1 gelöst. Fig. 5 zeigt die entwickelte Einrichtung in einem ersten Ausführungsbeispiel.

Bei der Lösung dieser Aufgabe sind zwei Einflußgrößen mit gegenläufigen Auswirkungen zu berücksichtigen.

Einerseits muss die Dicke der Einrichtung minimiert werden, um sich an die Geometrie des Auges anpassen zu können; andererseits soll sie eine bestimmte Dicke aufweisen, um ein Bild zu einem gesunden Bereich auf der Netzhaut verschieben zu können.

Das Hauptprinzip der vorliegenden Erfindung besteht darin, dass zur Verschiebung des Brennpunktes -der von einem oder mehreren konvexen Teilen der Linseneinrichtung erzeugt wirdvon einem funktionsbeeinträchtigten Bereich der Netzhaut zu einem funktionsfähigen Bereich, mehrere keilförmige Ausnehmungen in der Linseneinrichtung vorgesehen sind, wobei die keilförmigen Ausnehmungen geneigte Flächen aufweisen, die die Verschiebung eines oder mehrerer Brennpunkte bewirken.

Der Neigungswinkel der geneigten Flächen muss dabei nicht für alle geneigten Flächen der Ausnehmungen gleich sein.

Überraschenderweise wurde gefunden, dass eine deutliche Reduktion der Dicke der Linseneinrichtung dadurch erzielbar ist, dass mehrere keilförmige Ausnehmungen z.B. auf der Rückseite, d.h. der Netzhaut zugewandten Seite vorgesehen sind, z.B. in Form eines Fresnel-Prismas, wobei gleichzeitig eine ausreichende Verschiebung des Brennpunktes einstellbar bleibt.

Die unten aufgeführte Tabelle zeigt für bestimmte Brechungsindizes von möglichen Materialien (Spalte 1) den benötigten Prismenwinkel (Spalte 2) und vergleicht dann die zusätzliche Dicke, die eine Linsenvorrichtung aus dem Stand der Technik zu einer intraokularen Linse hinzufügt mit der erfindungsgemäßen Vorrichtung (Spalten 3 und 4). Die zusätzliche Dicke, die z.B. durch eine Fresnel-Linse verursacht wird, ist um einen Faktor N (N ist die Anzahl der Perioden) kleiner. Ist z.B. N = 10, dann wird die erfindungsgemäße Vorrichtung um einen Faktor 10 dünner. Die Linsenvorrichtung aus dem Stand der Technik ist plan-konvex, typischerweise 6 mm Durchmesser, und hat den Brennpunkt im Abstand a.

| Brechungsindex $n_1$ des optisehen Materials | Prismenwinkel $\varepsilon$ für eine Verschiebung von 1,0 mm (in Grad) | Zusätzliche Dicke bei einer aus dem Stand der Technik bekannten intraokularen Linse mit einem Durchmessers von 6,0 mm für ein gewöhnliches Prisma (in mm) | Zusätzliche Dicke einer Fresnel-Linse mit 10 Perioden (in mm) |
|---|---|---|---|
| 1,45 | 34,0 | 3,4 | 0,34 |
| 1,50 | 25,3 | 2,6 | 0,26 |
| 1,55 | 20, | 2,0 | 0,20 |
| 1,60 | 16,4 | 1,7 | 0,17 |

Der benötigte Prismenwinkel kann mittels der folgenden Formel berechnet werden (Es gibt natürlich auch andere Formeln bzw. Approximationen):

$$\arcsin\left(\frac{d}{a}\right) = \arcsin\left(\frac{n_1}{n_2}\sin\varepsilon\right) - \varepsilon \, ,$$

wobei

$\varepsilon$: Prismatischer Winkel (36)

d: Abstand von der Makula (22) zu dem gewünschten gesunden Punkt auf der Netzhaut (46)

a: Abstand von der Makula (22) zur hinteren Ebene der intraokularen Linse (30)

$n_1$: Brechungsindex des intraokularen Linsenmaterials

$n_2$: Brechungsindex des Kammerwassers/Glaskörpers (typischerweise 1,336).

In obiger Tabelle wurde die Formel numerisch gelöst. Als weitere typische Parameter wurden verwendet: d = 1,0 mm; a = 17,0 mm und $n_2$ = 1,336.

Die Dicke der intraokularen Linseneinrichtung wird durch die Auswahl bestimmter Materialien mit spezifischen Brechungsindizes kontrolliert, wobei der für eine bestimmte Bildverschiebung erforderliche Winkel z.B. der Fresnel-Prismen oder der keilförmigen Ausnehmungen kleiner ist, wenn der Brechungsindex höher ist.

Ein weiterer Vorteil der erfindungsgemäßen Einrichtung besteht darin, dass diese in einem Ausführungsbeispiel einstückig ausgebildet ist, wobei die Einrichtungen aus dem Stand der Technik mehrstückig ausgeführt sind, was zu Problemen bei der Implantierung und der Verträglichkeit der körperfremden Linseneinrichtungen führen kann, da mehrstückige Linseneinrichtungen meist nicht die zur Implantation gewünschte Faltbarkeit (große Operationswunde (Skleratunnel-Schnitt) nötig) und die gewünschte Verträglichkeit aufweisen.

Die Implantationsorte der intraokularen Linseneinrichtung können verschiedene Bereiche des Auges (Vorderkammer, Hinterkammer oder Kapsel der natürlichen Linse) sein. Je nach Implantationsort sind die Halterungsvorrichtungen entsprechend nach dem Stand der Technik anzupassen.

**[Beispiele]**

Ausführungsbeispiele, die teilweise nur zum Verständnis der Erfindung beitragen, teilweise aber auch unter den Schutzbereich der Erfindung fallen, sind in den Zeichnungen 1 bis 8 und der weiteren Beschreibung dargestellt:

- Fig.1 stellt das Auge des Menschen in Schnittansicht mit einer Linseneinrichtung (30) in einer ersten Ausführungsform dar.
- Fig.2 stellt eine Detailansicht von Fig. 1 dar, umfassend ferner eine Schutzschicht (34).
- Fig.3 stellt ein Ausführungsbeispiel mit einem glatten Segment auf der zur Makula (22) gewandten Seite der Einrichtung dar.
- Fig.4 zeigt ein weiteres Ausführungsbeispiel mit zwei unterschiedlichen Neigungswinkeln (36, 37).
- Fig.5 zeigt ein erfindungsgemässes Ausführungsbeispiel, bei dem als konvexer Teil der Linseneinrichtung die Segmente einer Fresnel-Linse (48) im vorderen Teil der Linseneinrichtung und die keilförmigen Ausnehmungen (32) im hinteren, der Makula zugeordneten Teil, der Linseneinrichtung vorgesehen ist. Die Linseneinrichtung wird durch die dicken durchgezogenen Linien dargestellt.
- Fig.6 und 7 zeigen eine weitere vorteilhafte Ausführungsform, bei der die Linseneinrichtung durch eine Wand in zwei getrennte Kammern unterteilt ist.

  - Die erste Kammer, d.h. der vordere Teil der Linseneinrichtung ist aus elastischem transparentem Material ausgelegt, so dass die Krümmung und damit die Brennweite des konvexen Teils variierbar ist.
  - Die zweite Kammer ist so ausgebildet, dass der transparente Träger der keilförmigen Ausnehmungen gegen diese drehbar angeordnet ist, wobei die Drehung gegen die Vorspannung eines elastischen Elementes erfolgt. Somit kann die Neigung der schrägen Flächen der Ausnehmungen variiert werden. Die Drehung kann durch einen eigenen Antrieb direkt im Drehelement (49) erfolgen oder durch Zuführung eines transparenten Fluides (68) in die zweite Kammer erfolgen, so dass durch den erhöhten Innendruck in der Kammer oder einer darin angebrachten Hülle, Druck auf das drehbare Trägerelement der Ausnehmungen ausgeübt wird.

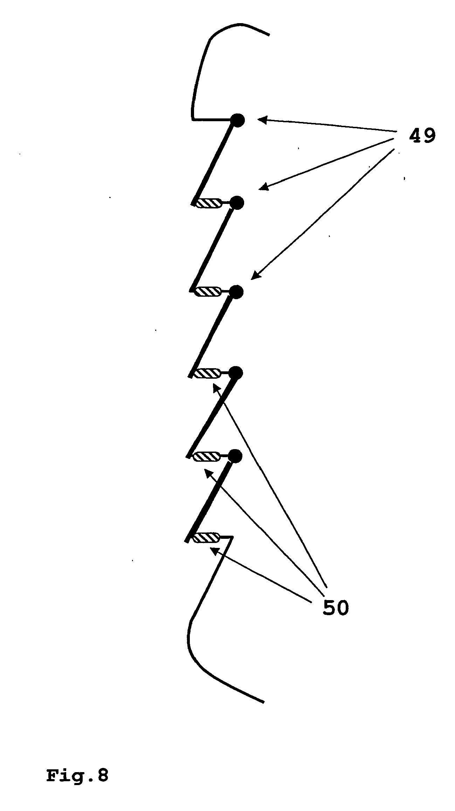

- Fig.8 zeigt einen Ausschnitt einer weiteren vorteilhaften Ausführungsform, bei der die keilförmigen Ausnehmungen jeweils eine drehbare, bewegliche, transparente und geneigte Fläche aufweisen. Jeder dieser drehbaren geneigten Flächen ist ein elastisches Element zugeordnet, welches gegen die Drehung vorgespannt ist.

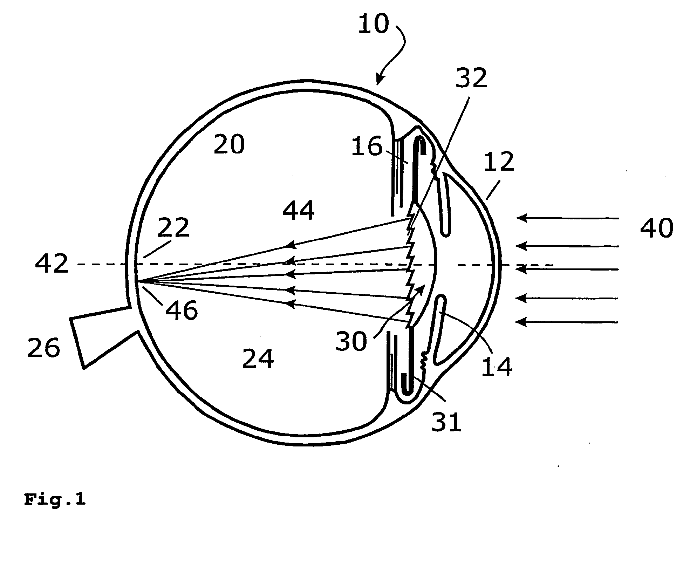

Fig. 1, zeigt ein menschliches Auge im Schnittbild (10) mit einer Linseneinrichtung (30). Parallel zur Symmetrieachse (42) der Linseneinrichtung einfallendes Licht (40) wird durch den vorderen konvexen Teil der Linseneinrichtung in einem Brennpunkt gebündelt und durch die geneigten Flächen der keilförmigen Ausnehmungen (32) in einen neuen Brennpunkt (46) auf die gesunde Netzhaut (20) bzw. Makula (22) verschoben.

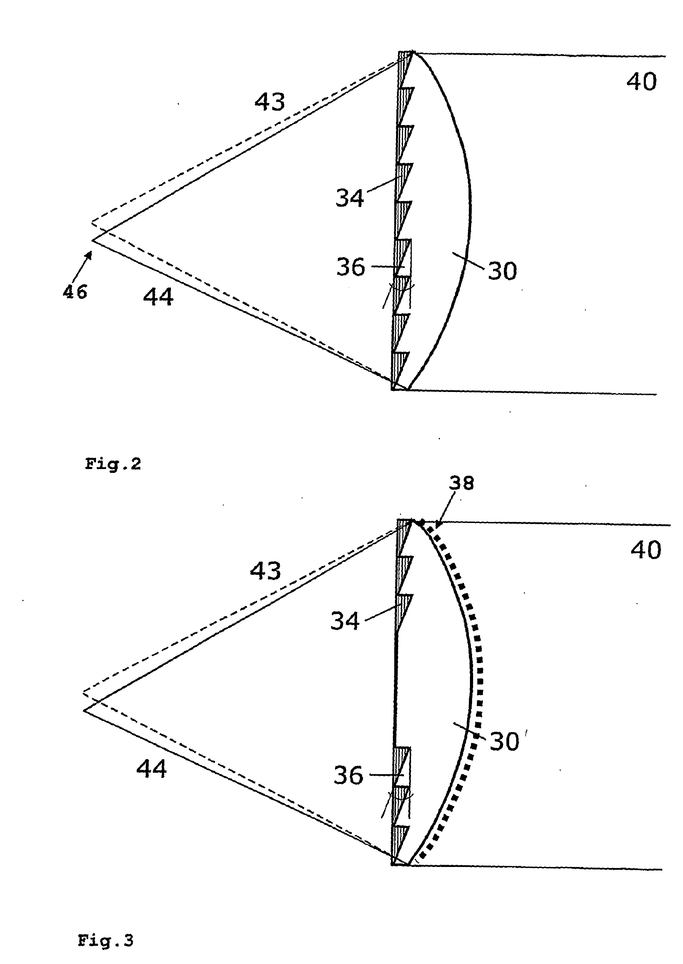

Fig. 2 zeigt die Einrichtung gemäß Fig. 1 jedoch mit einer Schutzschicht (34) zur Vermeidung

a) der Ablagerung von Partikeln oder Zellen in den Ausnehmungen (32)

b) von Reflexionen an der Linseneinrichtung nach Durchtritt durch diese und damit von Streulichteinfällen der Linseneinrichtung auf die Netzhaut, wozu die Schutzschicht (34) mit Antireflexionsmitteln versehen ist.

Dies erleichtert auch die Nachstar-Behandlung mit Hilfe von chirurgischen und/oder lasertechnischen Methoden. Deutlich zu erkennen ist der normale und der modifizierte Strahlengang (43,44). In diesem Beispiel weisen alle Ausnehmungen (32) geneigte Flächen mit dem gleichen Winkel (36) auf. Auf der von der Netzhaut abgewandten Seite ist ein UV-Schutz-Film oder Schicht (38) aufgetragen, da neueste Forschungsergebnisse gezeigt haben, dass die Eintrübung der Linse offenbar nicht nur krankhaft ist, sondern auch einen Schutz der dahinter gelegenen Netzhaut vor UV-Strahlung darstellt.

Fig. 3 zeigt ein weiteres Ausführungsbeispiel, bei dem im Bereich der optischen Symmetrieachse keine Ausnehmungen (32) vorgesehen sind, um die zentralen Lichtstrahlen ungehindert durchzulassen.

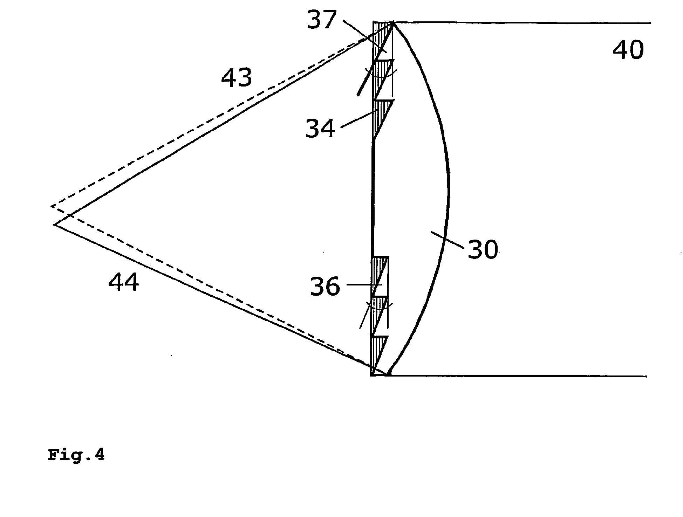

Fig. 4 zeigt das Ausführungsbeispiel gemäß Fig. 3, jedoch mit verschiedenen Neigungswinkeln (36, 37). Somit können verschiedene Bildteile verschieden stark verschoben werden.

Fig. 5 zeigt die erfindungsgemäße Linseneinrichtung (30), bei der an Stelle eines einseitig zumindest teilweise konvexen Linsen-Teils (33), mehrere konzentrische konvexe Segmente (48) einer Fresnel-Linse vorgesehen sind und die keilförmigen Ausnehmungen auf der gegenüberliegenden, der Makula (22) zugewandten Seite angebracht sind (Die Periode der Fresnel-Linse und der Prismen kann dabei unterschiedlich voneinander sein).

Gegenüber einer durchgängigen, einseitig konvexen Linse oder Teilen davon kann so nochmals die Dicke der Linseneinrichtung reduziert werden.

Eine besonders bevorzugte Ausführungsform (nicht dargestellt) ist dadurch erzielbar, dass die der Makula zugewandte Seite der Linseneinrichtung eben ausgeführt ist und die andere Seite solche geformte Segmente aufweist, wie sie sich ergeben, wenn man zu den konzentrischen konvexen Segmenten (48) einer Fresnel-Linse die angeschrägten Keile der Prismeneinrichtung hinzuaddiert, und somit die Linsenwirkung und die Verschiebung des Brennpunktes in einem Fresnel-Element kombiniert. Diese vordere Seite ist in Fig. 5 dargestellt. Dadurch lässt sich die Dicke der Linseneinrichtung noch weiter reduzieren. Aus Fertigungsgründen ist es bei dieser Ausführungsform jedoch vorteilhaft, die Einrichtung zu verstärken und die Ausnehmungen der kombinierten Fresnel-Elemente aus einem ausreichend dicken Materialblock zu entnehmen.

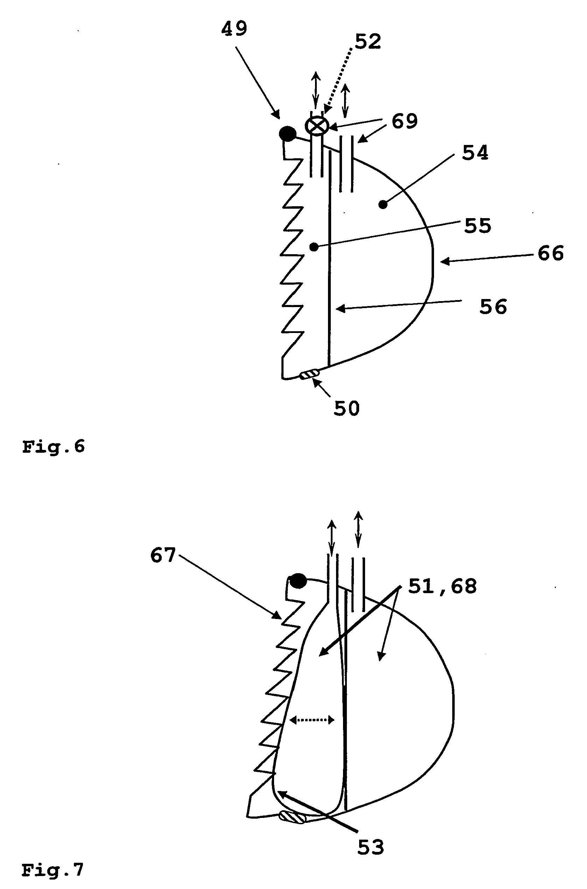

Fig. 6 zeigt eine Ausführungsform, in der im vorderen Teil der Linseneinrichtung, als Teil einer vorderen Kammer (54) ein in der Krümmung veränderbares konvexes Linsenelement vorgesehen ist. Dieses Linsenelement kann z.B. im Falle der Vorsehung von Pumpmitteln an der Linseneinrichtung, durch Zuführung oder Entnahme von z.B. Kammerflüssigkeit oder einer auf den Brechungsindex des Hüllenmaterials und/oder der Kammerflüssigkeit abgestimmten Flüssigkeit, oder durch Volumenveränderungsmittel in der Krümmung verändert werden.

Fig. 7 zeigt eine weitere ganz besonders bevorzugte Ausführungsform, bei der eine zweite, hintere Kammer (55), von der ersten (54) durch eine Wandung (56) getrennt vorgesehen ist. In dieser kann gemäß Fig. 7 eine elastische transparente Hülle (53) vorgesehen werden. Einen Teil dieser zweiten Kammer bildet ein transparentes Trägerelement (67) für mehrere keilförmige Ausnehmungen (32), welches gegen den ersten vorderen Linsenteil oder die vordere Kammer (54) durch ein Drehelement (49) drehbar gelagert ausgeführt ist. Ein elastisches Element ist mit dem Trägerelement (67) verbunden und gegen die Drehung des Elementes (67) vom restlichen Teil der hinteren Kammer (55) vorgespannt. Dabei ist zu vermeiden, dass die senkrechten Flächenstücke der Keile aus der parallelen Stellung zur optischen Achse herausrotiert werden, so dass diese nicht am "optischen Strahlengang" teilnehmen können.

Durch Zuführung oder Entnahme von z.B. Kammerflüssigkeit oder einer auf den Brechungsindex des Hüllenmaterials und/oder der Kammerflüssigkeit abgestimmten Flüssigkeit, z.B. durch die gleichen oder die selben Pumpmittel wie für die erste vordere Hülle, kann so das hintere Trägerelement, durch Volumenzunahme in einer in der Kammer (55) vorgesehenen elastischen Hülle (53), in der Neigung gegen die optische Achse veränderbar ausgeführt werden, so dass die veränderbare Neigung der keilförmigen Ausnehmungen (32) zu einer größeren oder kleineren Verschiebung des Brennpunktes führt.

Fig. 8 zeigt einen Teil einer weiteren Ausführungsform bei der in der zweiten, hinteren Kammer (55) keine elastische Hülle vorgesehen ist. Den keilförmigen Ausnehmungen ist jeweils eine bewegliche transparente und geneigte Fläche zugeordnet, welche durch Drehelemente (49) drehbar gelagert ist. Jeder dieser beweglichen geneigten Flächen ist ein elastische Element (50) zugeordnet. Die Drehung der beweglichen, geneigten Flächen erfolgt hierbei in dem Fall, dass durch die zugeordneten Pumpmittel der Innendruck der hinteren Kammer (55) erhöht wird. Gegenüber dem Ausführungsbeispiel in Fig. 7 ist hierbei vorteilhaft, dass eine Kompensation der Drehung der -im ungedrehten Zustand, waagerechten- Stücke, welche parallel zur optischen Achse verlaufen, nicht kompensiert werden muss.

Als Pumpmittel können z.B. nanostrukturierte Mittel eingesetzt werden, die das Phänomen der akustischen Oberflächenwellen, ggf. unterstützt durch Adhäsionskräfte ausnutzen. Solche Pumpmittel sind zur Zeit (Mai 2003) z.B. bei der Firma Advalytix AG in 85649 BRUNNTHAL erhältlich. Besonders vorteilhaft ist es hierbei solche Pumpmittel einzusetzen, deren Leistungsaufnahme ein Maß für den im Augeninneren herrschenden Druck darstellen. So könnte der Augeninnendruck über die Messung der Pumpleistung der Pumpmittel von aussen gemessen werden.

Als Energieversorgungsmittel für die Volumenveränderungs- oder Pump-Mittel können implantierte Batterien oder Empfänger und/oder Wandler für die Energiezufuhr von außerhalb des Körpers des Patienten, z.B. durch elektro- und/oder magnetische Felder vorgesehen werden. Als Medien zur Volumenveränderung kann neben der im Augapfel befindlichen Kammerflüssigkeit ein transparentes Medium, z.B. auch nicht flüssiges Medium eingesetzt werden, welches in seiner Brechzahl an die Brechzahl der Kammerwandungen und/oder elastischen Hülle (53) angepasst ist.

Wie dem Fachmann sofort ersichtlich ist, können in Kombination mit den keilförmigen Ausnehmungen oder konvexen Linsenteilen oder Segmenten einer Fresnel-Linse, weitere optische Mittel, wie z.B. Linsen zur Nahfeldkorrektur vorgesehen sein. Weitere optische Mittel können natürlich -aufgrund der erfindungsgemäß gewonnenen Reduktion der Dicke der Linseneinrichtung- im Strahlengang vor oder hinter der erfindungsgemäßen Linseneinrichtung angeordnet werden. Zudem kann die Fresnel-Linse selbst aufgrund ihres flexiblen Designs Zonen unterschiedlicher sphärischer oder auch nicht-sphärischer Krümmung aufweisen, um zusätzliche optische Effekte zu erzielen, z.B. zur Realisierung einer multifokalen Wirkung für gleichzeitiges Nah- und Weitsehen.

**[Bezugszeichenliste]**

| | |
|---|---|
| **10** | Auge |
| **12** | Hornhaut |
| **14** | Iris |
| **16** | Sulcus ciliaris |

| | |
|---|---|
| **20** | Netzhaut (Retina) |
| **22** | Makula |
| **24** | Glaskörper |
| **26** | Sehnerv |

| | |
|---|---|
| **30** | Linseneinrichtung oder intraokulare Linse |
| **31** | Haptik-Haken (Halterungsteile) |
| **32** | keilförmige Ausnehmungen |
| **33** | konvexer Linsenteil einer Standardlinse |
| **34** | Schutzschicht |
| **36** | Prismenwinkel |
| **37** | zweiter Prismenwinkel |
| **38** | UV-Schutz-Film |

| | |
|---|---|
| **40** | einfallendes Licht |
| **42** | Symmetrieachse der intraokularen Linse |
| **43** | normaler Strahlengang im Auge |
| **44** | fokussierter und abgelenkter Lichtstrahl |
| **46** | neuer, verschobener Brennpunkt auf der gesunden Netzhaut |
| **48** | Segmente einer Fresnel-Linse |
| **49** | Drehelement |

| | |
|---|---|
| **50** | elastisches Element |
| **51** | Volumenänderungsmittel |
| **52** | Pumpmittel |
| **53** | elastische, transparente Hülle |
| **54** | erste, vordere Kammer |
| **55** | zweite, hintere Kammer |
| **56** | Wandung |

| | |
|---|---|
| **66** | elastischer, konvexer Linsenteil |
| **67** | Trägerelement für Ausnehmungen (32) |
| **68** | transparentes Fluid |
| **69** | Zu-, Abfuhrkanäle |

**Patentansprüche**

1. Linseneinrichtung zur Behandlung von Sehbeeinträchtigungen umfassend ein Fixierungselement zum Befestigen dieser im Auge wobei diese mindestens ein konvexes Linsenelement und mehrere keilförmige Ausnehmungen (32) aufweist **dadurch gekennzeichnet, dass** die Linseneinrichtung auf einer Seite mehrere keilförmige Ausnehmungen und auf der anderen Seite eine Superposition von sphärischen und nicht-sphärischen Segmenten einer oder mehrerer Fresnel-Linsen (48) aufweist.

2. Linseneinrichtung nach Anspruch 1 **dadurch gekennzeichnet, dass** die konvexen Linsenelemente als Segmente einer Fresnel-Linse (48) ausgeführt sind.

3. Linseneinrichtung nach Anspruch 1 bis 2 **dadurch gekennzeichnet, dass** die keilförmigen Ausnehmungen (32) unterschiedliche Winkel (36, 37) aufweisen.

4. Linseneinrichtung nach Anspruch 1 bis 3 **dadurch gekennzeichnet, dass** die Rückseite eine Beschichtung oder Schutzschicht (34) als Auffüllung der Ausnehmungen aufweist, wobei die Schutzschicht (34) Antireflexionsmittel aufweist, welche die Reflexion von Licht an den Begrenzungen der Linseneinrichtung nach Durchtritt durch die Linseneinrichtung verhindert.

5. Linseneinrichtung nach Anspruch 1 bis 4 **dadurch gekennzeichnet, dass** die Beschichtung (34) und/oder das Material der Einrichtung einen Brechungsindex gleich dem der Kammerflüssigkeit aufweist.

6. Linseneinrichtung nach Anspruch 1 bis 5 **dadurch gekennzeichnet, dass** die Linseneinrichtung eine vordere und eine hintere Kammer (54, 55) aufweist, welche durch eine ebenfalls transparente Wandung (56) getrennt sind, wobei die von der Netzhaut abgewandte vordere Kammer mindestens ein konvexes, elastisches Element aufweist, so dass durch Einstellung der Krümmung dieses Elementes die Brennweite dieses Elementes variierbar ausgeführt ist.

7. Linseneinrichtung nach Anspruch 6 **dadurch gekennzeichnet, dass** die Linseneinrichtung in der hinteren, der Netzhaut zugewandten Kammer eine transparente elastische Hülle (53) aufweist und die hintere Kammer ein Trägerelement (67) für die Ausnehmungen (32) aufweist, welches gegen den Rest der hinteren Kammer drehbar gelagert ausgeführt ist, so dass der Neigungswinkel der geneigten Flächen der Ausnehmungen (32) verstellbar ist.

8. Linseneinrichtung nach Anspruch 7 **dadurch gekennzeichnet, dass** das Trägerelement mit einem gegen die Drehung vorgespannten elastischen Element (50) verbunden ist.

9. Linseneinrichtung nach Anspruch 6 bis 8 **dadurch gekennzeichnet, dass** jede Kammer (54, 55) mit einem Zu- und Abfuhrkanal (70) verbunden ist, welchem jeweils direkt oder vermittelt über ein Ventil oder mehrere Ventile mindestens ein Pump- oder Volumenveränderungsmittel (51, 52) zugeordnet ist, so dass bei Aktivierung der Pump- oder Volumenveränderungsmittel entweder das elastische, konvexe Element der vorderen Kammer (54) und/oder die elastische Hülle (53) eine Formveränderung erfährt und ausgelöst durch die Formänderung der Hülle (53) das Trägerelement (67) für die Ausnehmungen (32) eine Drehung erfährt.

10. Linseneinrichtung nach Anspruch 6 und 9 **dadurch gekennzeichnet, dass** in der hinteren, der Netzhaut zugewandten Kammer (55) den keilförmigen Ausnehmungen jeweils eine bewegliche transparente und geneigte Fläche zugeordnet ist, welche drehbar gelagert durch Drehelemente (49) ausgeführt sind, wobei jeder dieser drehbar geneigten Flächen ein elastisches Element (50) zugeordnet ist und im Falle des Rückbezugs auf Anspruch 9, bei Aktivierung der, der vorderen Kammer zugeordneten Pump- oder Volumenveränderungsmittel (51, 52) die geneigten Flächen eine Drehung erfahren, so dass der Brennpunkt auf der Netzhaut verschiebbar ausgeführt ist.

11. Linseneinrichtung nach Anspruch 1 und 10 **dadurch gekennzeichnet, dass** jede Kammer (54, 55) mit einem transparenten Medium, vorzugsweise fluiden Medium gefüllt ausgeführt ist, dessen Brechungszahl an die der Kammerflüssigkeit und/oder der transparenten, elastischen Hülle (53) und/oder der Wandung (56) angepasst ausgeführt ist.

12. Linseneinrichtung nach Anspruch 9 **dadurch gekennzeichnet, dass** als Energieversorgungsmittel für die Volumenveränderungs- oder Pump-Mittel implantierte Batterien oder Empfänger und/oder Wandler für die Energiezufuhr von ausserhalb des Körpers des Patienten, z.B. durch elektro- und/oder magnetische Felder vorgesehen sind.

13. Linseneinrichtung nach Anspruch 1 bis 12 **dadurch gekennzeichnet, dass** an Stellen ohne keilförmigen Ausnehmungen oder konvexen Linsenteilen oder Segmenten einer Fresnel-Linse, aber auch in Kombination mit diesen, weitere optische Mittel, wie z.B. Linsen zur Nahfeldkorrektur vorgesehen sind, wobei diese - aufgrund der erfisndungsgemäss gewonnenen Reduktion der Dicke der Linseneinrichtung - auch im Strahlengang vor oder hinter der erfindungsgemässen Linseneinrichtung vorgesehen sein können.

14. Linseneinrichtung nach Anspruch 1 bis 13 **dadurch gekennzeichnet, dass** die Linseneinrichtung mindestens einen UV-Schutz-Film (38) zum Schutz der Netzhaut vor UV-Strahlung aufweist.

15. Linseneinrichtung nach Anspruch 1 bis 14 **dadurch gekennzeichnet, dass** die Linseneinrichtung zur Verkleinerung des Skleratunnel-Schnittes faltbar oder elastisch ausgeführt ist.

16. Linseneinrichtung nach Anspruch 1 bis 15 **dadurch gekennzeichnet, dass** die Linseneinrichtung auf einer Seite plan und auf der anderen Seite eine Superposition von keilförmigen Ausnehmungen (32) und sphärischen und nichtsphärischen Segmenten einer oder mehrerer Fresnel-Linsen (48) aufweist.

17. Linseneinrichtung nach Anspruch 9 bis 15 soweit rückbezogen auf Anspruch 9 **dadurch gekennzeichnet, dass** die Pump- oder Volumenveränderungsmittel eine Kennlinie aufweisen, die aus der aufgenommenen Pump- oder Volumenänderungs-Leistung Rückschlüsse auf den Gegendruck erlaubt, gegen den die Pump- oder Volumenänderungsmittel arbeiten, so dass über die Messung der aufgenommenen Leistung eine Bestimmung des Druckes im Inneren des Auges möglich ist.

**Claims**

1. Lens appliance for visual disorder treatment comprising a fixing element to secure the same in the eye and showing at least one convex lens element and several wedge-shaped recesses (32), **characterized in that** the lens appliance has at one side several wedge-shaped recesses and on its other side a superposition of spherical and non-spherical segments of one or more Fresnel lenses.

2. Lens appliance according to Claim 1, **characterized in that** the convex lens elements are designed as segments of a Fresnel lens (48).

3. Lens appliance according to Claims 1 to 2, **characterized in that** the wedge-shaped recesses (32) have different angles (36, 37)

4. Lens appliance according to Claims 1 to 3, **characterized in that** the back has a coating or protective layer (34) as a filling of the recesses, with such protective layer (34) containing antireflection agents to prevent the reflection of light at the boundaries of the lens appliance at passing the lens appliance.

5. Lens appliance according to Claims 1 to 4, **characterized in that** the coating (34) and/or the material of the appliance has the same refractive index as the chamber liquor.

6. Lens appliance according to Claim 1, **characterized in that** the lens appliance consists of a front and a back chamber (54, 55) that are separated by an also transparent wall, with the front chamber situated away from the retina having at least one convex elastic element so that by adjustment of the curvature of this element the focal length of this element is variable.

7. Lens appliance according to Claim 6, **characterized in that** the lens appliance has in its back chamber positioned towards the retina a transparent elastic enclosure (53), and that the back chamber has a support element (67) for the recesses (32) that is mounted pivotably against the remaining part of the back chamber so that the inclination angle of the inclined surfaces of the recesses (32) is adjustable.

8. Lens appliance according to Claim 7, **characterized in that** the support element is connected to an elastic element (50) prestressed against its turn.

9. Lens appliance according to Claims 6 to 8, **characterized in that** each of the chambers (54, 55) has a inlet-outlet channel (70) to each of them is associated directly or by a valve or several valves at least one pumping- or volume-modifying medium 51, 52) so that with activating the pumping- or volume-modifying medium either the elastic convex element of the front chamber (54) and/or the elastic enclosure (53) is deformed, with such deformation of the enclosure (53) causing a turn of the support element (67) for the recesses (32).

10. Lens appliance according to Claims 6 and 9, **characterized in that** in the back chamber (55) positioned towards the retina one moveable transparent and inclined surface is associated to each of the wedge-shaped recesses that are mounted pivotably
by pivoting elements (49), and that to each of these pivotably inclined surfaces an elastic element (50) is associated and, when relating to Claim 9, at the activation of the pumping- or volume-modifying medium (51, 52) a turn of the inclined surfaces is caused so that the focus is arranged moveably on the retina.

11. Lens appliance according to Claims 1 and 10, **characterized in that** each of the chambers (54, 55) is designed filled with a transparent medium, preferably a fluid medium, whose refractive index adapts to that of the chamber liquor and/or of the transparent elastic enclosure (53), and/or of the wall (56).

12. Lens appliance according to Claim 9, **characterized in that** as an energy supply means for the volume-modifying- or pumping medium implanted batteries or receivers and/or transducers for energy supply from outside the patient's body, e. g. by electric or magnetic fields are provided for.

13. Lens appliance according to Claims 1 to 12, **characterized in that** at regions without wedge-shaped recesses or at the convex lens sections or segments of a Fresnel lens, but also in combination with them, additional optical means as for example lenses for near field correction are provided for that may - by virtue of the reduced thickness of the lens appliance received according to the invention - also be placed in the ray trace before or behind the lens appliance according the invention.

14. Lens appliance according to Claims 1 to 13, **characterized in that** the lens appliance has at least one UV-protective film (38) to protect the retina from UV-radiation.

15. Lens appliance according to Claims 1 to 14, **characterized in that** the lens appliance is pliable or elastic in order to minimize the sclera tunnel incision.

16. Lens appliance according to Claims 1 to 15, **characterized in that** the lens appliance is plane on one side and has on its other side a superposition of wedge-shaped recesses (32) and spherical and non-spherical segments of one or more Fresnel lenses (48).

17. Lens appliance according to Claims 9 to 15, as far as related to Claim 9 **characterized in that** the pumping- and volume-modifying means have an identification line that allows to deduce from the taken pumping- and volume-modifying performance the counter-pressure against which the pumping- or volume-modifying means work, so that the measuring of the taken performance enables to determine intraocular tension.


**Revendications**

1. Dispositif de lentille pour le traitement des troubles de vision comprenant un élément de fixation pour l'attacher dans l'oeil et montrant au moins un élément de lentille et plusieurs creux en forme de clavette (32), **caractérisé en ce que** le dispositif de lentille à sur un côté plusieurs creux en forme de clavette et sur son autre côté une superposition de segments sphériques ou non-sphériques d'une ou plusieurs lentilles de Fresnel (48).

2. Dispositif de lentille selon la Revendication 1, **caractérisé en ce que** les éléments de lentille convexes sont exécutés comme segments d'une lentille de Fresnel (48).

3. Dispositif de lentille selon les Revendications 1 à 2, **caractérisé en ce que** les creux en forme de clavette (32) ont des angles différents (36, 37).

4. Dispositif de lentille selon les revendications 1 à 3, **caractérisé en ce que** le dos porte un revêtement ou une couche protectrice (34) garnissant les creux, cette couche protectrice (34) contenant des agents antireflets qui empêchent le reflet de la lumière aux bords du dispositif de lentille après son passage par le dispositif de lentille.

5. Dispositif de lentille selon la Revendication 1, **caractérisé en ce que** le revêtement (34) et/ou le matériel du dispositif est d'un index de réfraction pareil à celui du liquide de chambre.

6. Dispositif de lentille selon les Revendications 1 à 5, **caractérisé en ce que** le dispositif de lentille a une chambre avant et une chambre derrière (54, 55) qui sont séparées par une paroi également transparente (56), la chambre avant détachée de la rétine ayant au moins un élément élastique convexe tel que par réglage de la courbure de cet élément la distance focale de cet élément est variable.

7. Dispositif de lentille selon la Revendication 6, **caractérisé en ce que** le dispositif de lentille a dans la chambre derrière orientée vers la rétine une gaine transparente et élastique (53) et que la chambre derrière a un élément portatif (67) pour les creux (32) qui est monté de manière pivotante vis-à-vis de la partie restante de la chambre derrière, de sorte que l'angle d'inclinaison des surfaces inclinées des creux (32) est ajustable.

**8.** Dispositif de lentille selon la Revendication 7, **caractérisé en ce que** l'élément portatif est relié à un élément élastique (50) précontraint contre sa rotation.

**9.** Dispositif de lentille selon les Revendications 6 à 8, **caractérisé en ce que** chaque chambre (54, 55) a une manche d'entrée et d'évacuation (70) à chacune de celles-ci est attribué directement ou par intermédiaire d'une seule ou plusieurs soupapes au moins un médium pompeur ou modificateur-volume (51, 52) de sorte que par activation du médium pompeur ou modificateur-volume soit l'élément élastique convexe de la chambre avant (54) soit/et la gaine élastique (53) est déformé, une telle déformation de la gaine (53) causant un mouvement rotatif de l'élément portatif (67) pour les creux (32).

**10.** Dispositif de lentille selon les Revendications 6 et 9, **caractérisé en ce que** dans la chambre derrière orientée vers la rétine (55) une surface inclinée transparente et mouvable est attribuée à chaque creux en forme de clavette monté de manière pivotante par des éléments pivotants (49), et qu'à chacune de ces surfaces inclinées pivotantes est attribué un élément élastique (50) et, référence faite à la Revendication 9, que l'activation du médium pompeur ou modificateur-volume (51, 52) attribué à la chambre avant cause un mouvement tournant des surfaces inclinées qui arrange le foyer d'une manière mouvante sur la rétine.

**11.** Dispositif de lentille selon les Revendications 1 et 10, **caractérisé en ce que** chaque chambre (54, 55) est exécutée remplie d'un médium transparent, de préférence un médium fluide dont l'indice de réfraction s'adapte à celui-ci de la liquide de chambre et/ou de la gaine transparente et élastique (53) et/ou de la paroi (56).

**12.** Dispositif de lentille selon la revendication 9, **caractérisé en ce que** comme moyen d'alimentation en énergie pour le médium pompeur ou modificateur-volume sont prévus des accumulateurs ou récepteurs et/ou des transducteurs pour l'alimentation en énergie de l'extérieur du corps du patient, par exemple par des champs électriques et/ou magnétiques.

**13.** Dispositif de lentille selon les Revendications 1 à 12, **caractérisé en ce que** pour des régions sans des creux en forme de clavette ou pour les sections de lentille convexes ou les sections d'une lentille de Fresnel, mais aussi en combinaison avec ceux-ci, d'autres moyens optiques sont prévus comme par exemple des lentilles de correction de champs proche, qui - grâce à la réduction d'épaisseur du dispositif de lentille reçue selon l'invention - peuvent même être placés dans la marche des rayons avant ou derrière le dispositif de lentille selon l'invention.

**14.** Dispositif de lentille selon les revendications 1 à 13, **caractérisé en ce que** le dispositif de lentille a au moins un film protecteur U. V. (38) pour protéger la rétine du rayonnement ultraviolet.

**15.** Dispositif de lentille selon les Revendications 1 à 14, **caractérisé en ce que** le dispositif de lentille est pliable ou élastique pour minimiser l'incision tunnel de la sclérotique.

**16.** Dispositif de lentille selon les Revendications 1 à 15, **caractérisé en ce que** le dispositif de lentille est plan d'une de ses côtés et porte sur l'autre côté une superposition des creux en forme de clavette (32) et de segments sphériques et non-sphériques d'une ou plusieurs lentilles de Fresnel (48).

**17.** Dispositif de lentille selon les Revendications 9 à 15, en tant que faisant référence à la Revendication 9, **caractérisé en ce que** les médiums pompeurs ou modificateurs-volume monte une ligné d'identification permettant de conclure du rendement de pompage ou de modification de volume pris à la contre-pression contre laquelle les médiums pompeurs ou modificateurs-volume agissent, de sorte que par le mesurage du rendement pris il est possible de déterminer la pression intraoculaire.

Fig.1

Fig.2

Fig.3

EP 1 675 530 B1

Fig.4

13

48

32

33

Fig.5

**Fig.6**

**Fig.7**

Fig.8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4581031 A
- EP 0897293 B1
- DE 19751503 A1
- US 5089023 A